# EUROPÄISCHE PATENTSCHRIFT

(11) **EP 0 572 887 B1**
(45) Veröffentlichungstag und Bekanntmachung des Hinweises auf die Patenterteilung: **17.11.1999**
(21) Anmeldenummer: 93108338.0
(22) Anmeldetag: 24.05.1993
(51) Int. Cl.: C07D 249/06, G03C 7/305

(54) **Verfahren zur Herstellung von naphtholischen 2-Äquivalentcyankupplern**
Method of production of naphtholic two-equivalent cyan couplers
Procédé de préparation de copulants cyan naphtoliques à deux équivalents

(30) Priorität: 03.06.1992 DE 4218308
(43) Veröffentlichungstag der Anmeldung: 08.12.1993
(73) Patentinhaber: Agfa-Gevaert AG, 51373 Leverkusen (DE)
(72) Erfinder: Bergthaller, Peter, W-5060 Bergisch Gladbach 2 (DE)

(56) Entgegenhaltungen:
- EP-A- 0 401 612
- DE-A- 1 800 420
- DE-A- 3 209 486
- FR-A- 2 222 674
- US-A- 3 733 201

## Beschreibung

Die Erfindung betrifft ein Verfahren zur Herstellung von 2-Äquivalentkupplern. Insbesondere betrifft die Erfindung ein Verfahren zur Herstellung von als 2-Äquivalentkuppler geeigneten Verbindungen der Formel I 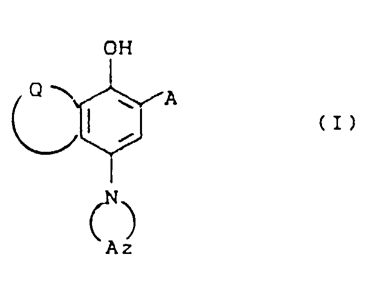 worin bedeuten
- A: eine Gruppe mit Elektronenakzeptorcharakter; z.B. Carbamoyl, Sulfamoyl oder Acylamino;
- Az: einen Rest zur Vervollständigung eines 5-gliedrigen heteroaromatischen Ringes (Azolring), an den ein 5-oder 6-gliedriger carbocyclischer oder heterocyclischer Ring ankondensiert sein kann;
- Q: einen Rest zur Vervollständigung eines ankondensierten Benzol- oder Pyridinringes.

2-Äquivalentkuppler haben im Aufbau farbfotografischer Materialien aus verschiedenen Gründen große technische Bedeutung erlangt: sie ermöglichen die Einsparung von Silberhalogenid durch geringeren Bedarf an Oxidationsäquivalenten, sie ermöglichen durch Wahl der Fluchtgruppe die Einstellung einer optimalen Kupplungskinetik, sie kuppeln in vielen Fallen mit größerer Einheitlichkeit zu den gewünschten Azomethin- oder Indophenolfarbstoffen, wodurch sich farbreinere Bilder erzeugen lassen und sie ermöglichen über die bei der Kupplung freigesetzte Fluchtgruppe, die gegebenenfalls entwicklungsaktivierende oder entwicklungshemmende Eigenschaften aufweisen kann, eine zusätzliche Steuerung des Entwicklungsprozesses, z.B. über sogenannte Interimage- oder Kanteneffekte.

Eine besondere Bedeutung kommt unter den Fluchtgruppen den heterocyclischen Fluchtgruppen mit einem oder mehreren N-Atomen als nukleophilen Zentren zu, insbesondere den monocyclischen oder bicyclischen Triazolen. Die Einführung von heterocyclischen Azolen in Gelbkupplerstrukturen aus der Reihe der Acylacetanilide wird gewöhnlich durch Umsetzung der halogenierten Kuppler mit der Fluchtgruppe in einer nukleophilen Substitution vorgenommen, vergl. beispielsweise EP-A-0 401 612, Seite 16. In DE-A-32 09 486, Seiten 90 und 91, wird die "Synthese eines Kupplers der Formel (III)" envähnt, worin ein Benzotriazolrest über ein Ringstickstoffatom in 1-Stellung an die Kupplungsstelle eines Kupplerrestes A₁ gebunden ist. Nach der angegebenen Definition (Seite 35) bedeutet A₁ einen Gelbkupplerrest einen Purpurrotkupplerrest oder einen im wesentlichen keine Farbe bildenden Kupplerrest.

In den bekannten und technisch genutzten Cyankupplerklassen, den Phenolen und den Naphtholen, ist ein Austausch von Halogen gegen heterocyclische Fluchtgruppennukleophile mit einem N-Atom nach dem Stand der Technik nicht möglich, weil das Halogenatom eine starke Stabilisierung über das aromatische System erfährt.

Cyan-DIR-Kuppler, bei denen der Inhibitor über ein Ring-N-Atom unmittelbar an die Kupplungsstelle gebunden ist, sind beispielsweise in FR-A-2 222 674 durch die allgemeine Formel III beschrieben, waren aber bisher wegen fehlender Synthesemöglichkeiten nicht verfügbar.

In beschränktem Maß können heterocyclische Azole durch ringsynthetische Maßnahmen, z.B. über 1,3-dipolare Cycloadditionen oder - im Fall der Benzotriazole - durch Modifizierung von Azofarbstoffstrukturen in Naphthole eingeführt werden, vergl. beispielsweise DE-A-1 800 420. Ein besseres Verfahren für die Einführung von Benzotriazolen in die Kupplungsstelle von naphtholischen Cyankupplern ist erst in letzter Zeit bekannt geworden (Angewandte Chemie 103/12 (1991), Seiten 1742-3). Es beruht auf der Venvendung von Selenverbindungen als Umpolungsreagentien, bevorzugt in der 4-wertigen Form, die sich mit den Kupplern in einer der Friedel-Crafts-Synthese nahestehenden Reaktion umsetzen und deren Reaktionsprodukte zu 2-Äquivalentkupplern umgesetzt werden können. Triebkraft der Reaktion scheint die energetisch günstige Freisetzung von elementarem Selen, bzw. Tellur zu sein.

Ein gewichtiger Einwand gegen die Verwendung von Se(IV)-Verbindungen, z.B. von Selendioxid, Ditosyl-selendiimin, Dichlorselen-N-tosylimin, Selenoxychlorid oder Selentetrachlorid, aber auch gegen den Einsatz von Tellur(IV)-Verbindungen als Umpolungsreagentien für die nukleophile Kupplungsstelle ist jedoch ihre hohe Toxizität, die durch den Einbau in organische Verbindungen und die dadurch erhöhte Gewebeverträglichkeit nicht vermindert, in kritischen Fällen sogar noch gesteigert wird. Die Verwendung von Se(IV)- und Te(IV)-Verbindungen ist insbesondere dann nicht angezeigt, wenn nach dem Ende der Umsetzung das eingesetzte Se oder Te nicht weitgehend vollständig als Element ausgefällt und abgetrennt werden kann.

Daher besteht für die Cyankuppler nachwievor ein Bedarf nach einem technisch unkomplizierten Syntheseverfahren das den nachträglichen Einbau einer heterocyclischen Fluchtgruppe in einen 4-Äquivalentcyankuppler ermöglicht.

Überraschenderweise wurde gefunden, daß einfache Umsetzungen an naphtholischen Cyankupplern mit einem Iod-Atom als Fluchtgruppe zu 2-Äquivalentkupplern mit heterocyclischen Azolfluchtgruppen führen, wenn diese durch Oxidation in Hetero-Iodinane übergeführt werden und mit heterocyclischen Azolen - bevorzugt in Gegenwart von Basen - umgesetzt werden.

Gegenstand der Erfindung ist ein Verfahren zur Herstellung einer Verbindung der Formel I 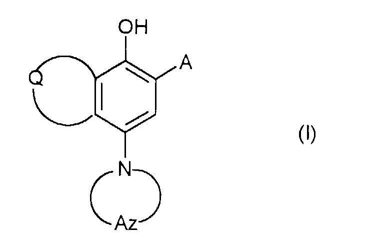 worin bedeuten
- A: eine Carbamoylgruppe, eine Sulfamoylgruppe oder eine Acylaminogruppe;
- Az: einen Rest zur Vervollständigung eines 5-gliedrigen heteroaromatischen Ringes (Azolring), an den ein 5- oder 6-gliedriger carbocyclischer oder heterocyclischer Ring ankondensiert sein kann;
- Q: einen Rest zur Vervollständigung eines ankondensierten Benzol- oder Pyridinringes,
dadurch gekennzeichnet, daß eine Verbindung der Formel II 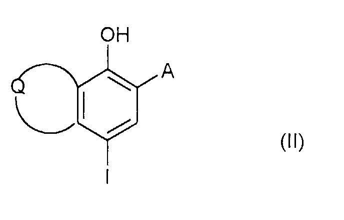 worin
- A und Q: die angegebene Bedeutung haben,
mit einem Acyloxygruppen übertragenden Oxidationsmittel oxidiert und anschließend mit einer Verbindung der Formel III umgesetzt wird 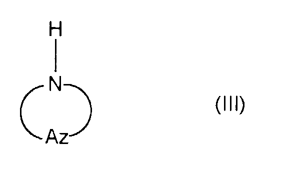 worin
- Az: die angegebene Bedeutung hat.

Die durch A dargestellten Carbamoylgruppe, Sulfamoylgruppen oder Acylaminogruppen können Ballastreste enthalten, z.B. langkettige Alkylreste und/oder Phenyl- oder Phenoxygruppen, die beispielsweise mit Alkyl-, Alkoxy-, Alkoxycarbonyl-, Alkylamino-, Acylamino-, Sulfonamide-, Carbamoyl- und/oder Sulfamoylgruppen substituiert sein können.

Ein durch Q vervollständigter ankondensierten Benzol- oder Pyridinring kann beispielsweise durch Acylamino-, Sulfonamido-, Alkoxycarbonylamino-, Amino- oder Hydroxylgruppen substituiert sein.

Der durch Az vervollständigte heteroaromatische Ring ist insbesondere ein 5-gliedriger, mindestens 2, vorzugsweise mindestens 3 Stickstoffatome enthaltender heteroaromatischer Ring (z.B. 1,2,3-Triazol oder 1,2,4-Triazol), an den gegebenenfalls ein weiterer aromatischer oder heteroaromatischer Ring ankondensiert sein kann (z.B. Benzotriazol).

Verbindungen der Formel II sind bekannt, z.B. aus US 3 642 485, US 3 790 384, DE-A-22 47 496. Ihre Herstellung erfolgt zweckmäßig aus dem entsprechenden 4-Äquivalentkuppler durch Umsetzung mit elementarem Iod oder mit Iodmonochlorid, wobei Friedel-Crafts-Katalysatoren im allgemeinen nicht benötigt werden.

Als Acyloxygruppen übertragende Oxidationsmittel lassen sich insbesondere die höherwertigen Acetate oder Trifluoracetate von Blei, Thallium, Mangan einsetzen, aber auch andere Oxidationsmittel, z.B. die Iodbenzolsulfonate oder -trifluoracetate ("Kosers reagent", J. Org. Chem. 49 (1984), 4700; bzw. "Zefirov's reagent", J. Org. Chem. 54 (1989), 2609). Chlorierende Oxidationsmittel sind weniger geeignet, weil sie bevorzugt zum chlorierten 2-Äquivalentkuppler führen.

Zu den Verbindungen der Formel III, die mit Hilfe des neuen Verfahrens als Fluchtgruppe in naphtholische 4-Äquivalentkuppler eingeführt werden können, zählen unter den monocyclischen Heteroaromaten die Imidazole, 1,2,3-Triazole, 1,2,4-Triazole, Tetrazole, Triazolone; unter den bicyclischen Verbindungen die Benzotriazole, Thienotriazole, Furotriazole, Tetrahydrobenzotriazole.

In den Verbindungen der Formel I ist die Gruppe 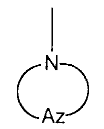 vorzugsweise eine Gruppe der Formel 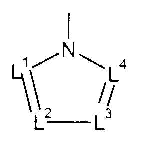 worin zwei der Ringglieder (L¹, L², L³, L⁴) für ein N-Atom und die beiden anderer für eine Gruppe 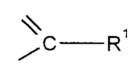 stehen,
worin R¹ für H, Alkyl, Alkylthio, Aryl, eine heterocyclische Gruppe (z.B. Furan oder Thiazol) oder -COOR² (mit R² = Alkyl oder Aryl) steht und worin, falls L¹ und L² für 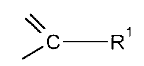 stehen,
die beiden Reste R¹ einen ankondensierten Benzolring bilden können.

Die Umsetzung der durch Oxidation aus den Verbindungen der Formel II erhaltenen Heteroiodinane mit den Verbindungen der Formel III wird zweckmäßigerweise in Lösung und in Gegenwart von Basen vorgenommen. Als Lösungsmittel eignen sich beispielsweise Dichlormethan, Ethylacetat, Dioxan, Tetrahydrofuran,1,2-Dimethoxymethan, Chlorbenzol, Pyridin.

Geeignete Basen sind beispielsweise starke organische Basen vom Amidin- oder Guanidintyp, z.B. Tetramethylguanidin, Diazabicyclononan, Diazabicycloundecan, aber auch peralkylierte Triamino-imino-phosphorane.

Nach dem erfindungsgemäßen Verfahren lassen sich beispielsweise die nachfolgend genannten Verbindungen herstellen.

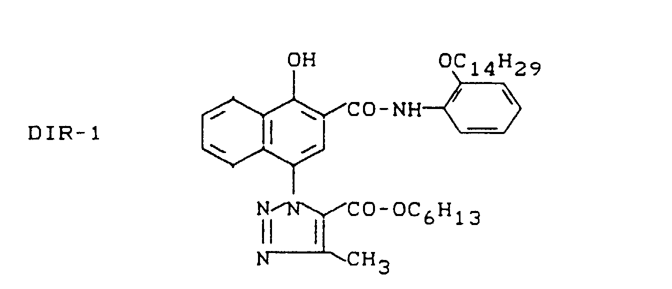

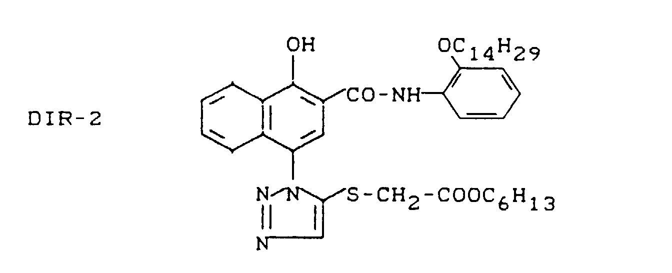

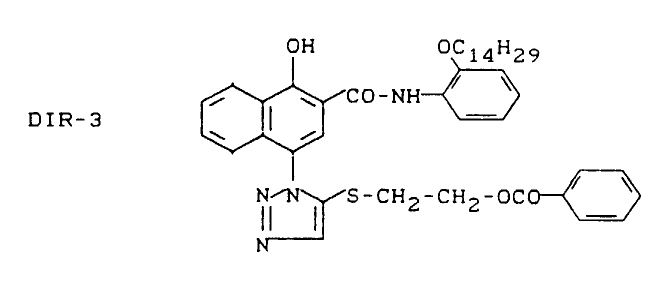

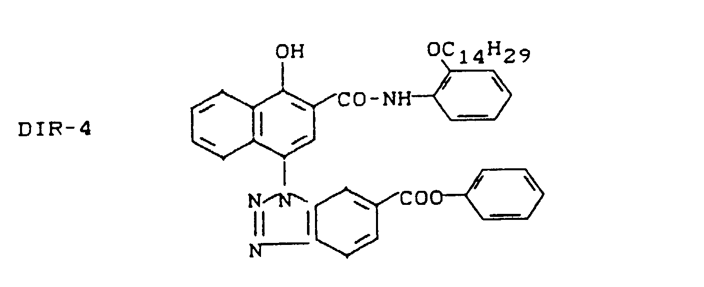

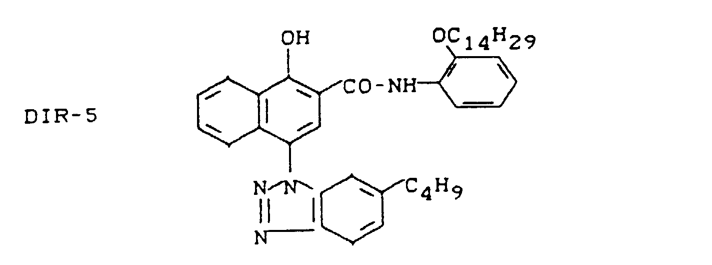

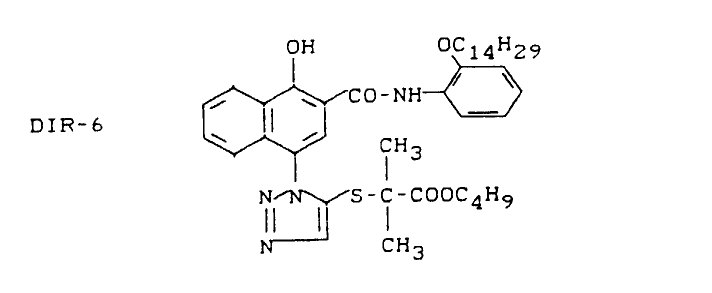

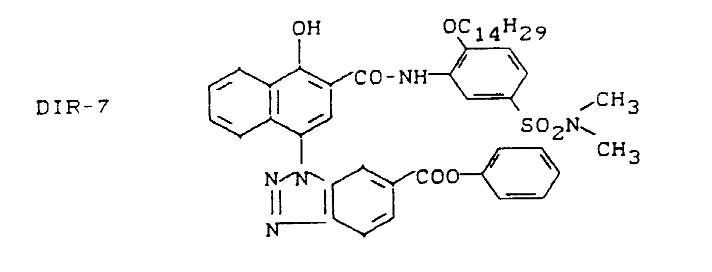

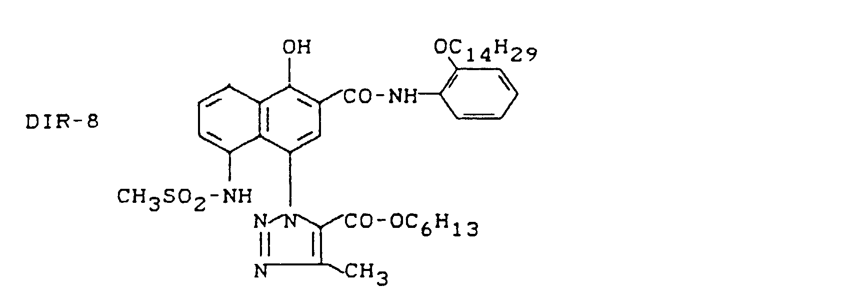

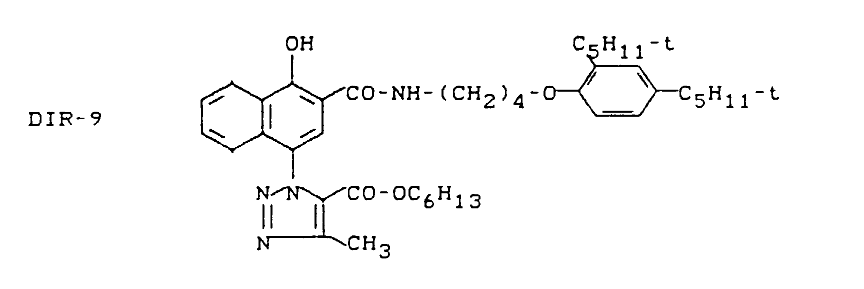

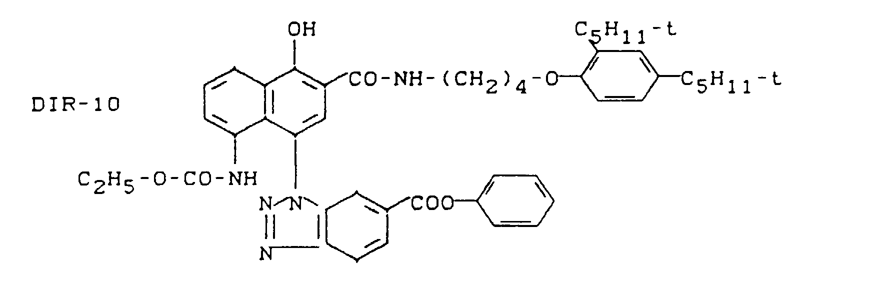

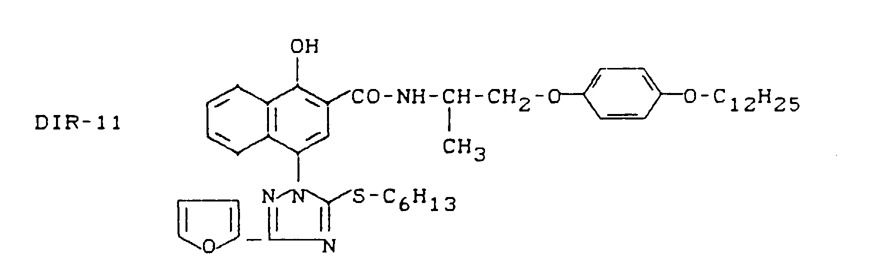

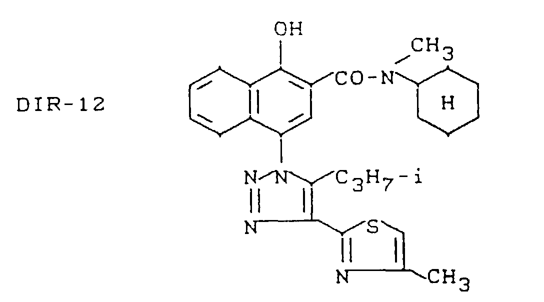

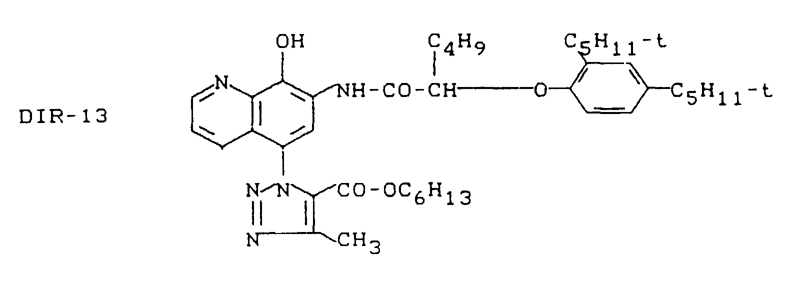

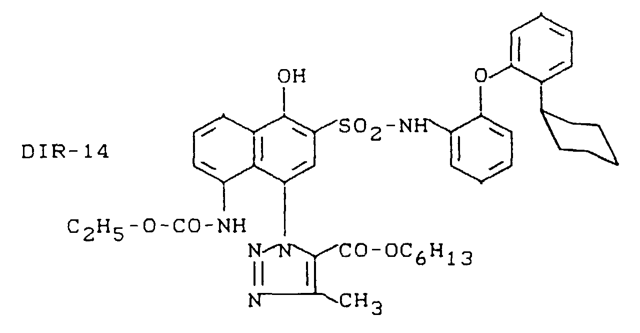

In den obigen Formeln sind die Cyan-DIR-Kuppler dargestellt mit einer Bindung zwischen der Kupplungsstelle des Kupplers und einen der drei N-Atome des Triazolringes. Mit der gewählten Darstellungsweise wird kein Anspruch darauf erhoben, daß die Formeln die tatsächlichen Verhältnisse richtig wiedergegeben. Der Triazolring kann auch über eins der beiden anderen N-Atome an die Kupplungsstelle gebunden sein, oder es kann sich um Isomerengemischehandeln.

Das Verfahren der vorliegenden Erfindung wird durch folgendes Synthesebeispiel erläutert:
1.1 Verbindung DIR-1
   Man setzt 4,75 g (10 mmol) 1-Naphthol-2-carbonsäure-(2-tetradecyloxy)anilid in 70 ml Pyridin mit 2,6 g Iod unter Rühren über 5 h bei Raumtemperatur um. Der iodierte Kuppler kristallisiert beim Einrühren der Lösung in 200 ml 10 %ige Salzsäure aus. Ausbeute 4,4 g. Schmelzpunkt: 72 bis 74°C.
1.2 Verbindung DIR-1
   Man löst 3 g (5 mmol) iodierten Kuppler aus 1.1 in 200 ml Dichlormethan und gibt unter Rühren 1,55 g (5 mmol) Iodbenzol-bistrifluoracetat zu. Das Gemisch färbt sich dunkel. Nach Stehen über 1 h und Zugabe von 1,2 g 5-Methyl-1,2,3-triazol-4-carbonsäure-n-hexylester und 1,2 g Tetramethylguanidin wird die Lösung 10 h unter Feuchtigkeitsausschluß stehengelassen. Man gibt 20 ml 10 %iger Salzsäure zu, trennt die Phasen und wäscht die Dichlormethanphase mit Wasser. Nach Trocknen über Natriumsulfat wird eingedampft, in Toluol aufgenommen und über 100 g Kieselgel mit Cyclohexan-Toluol-Ethylacetat-Gemischen bei steigendem Anteil Toluol und Ethylacetat chromatographiert.

Nach Abtrennung zweier verlaufender Flecke werden aus den polaren Eluaten 1,2 g Verbindung DIR-1 mit dem Schmelzpunkt 82 bis 84°C (aus Cyclohexan) erhalten.

¹H-NMR (200 Mhz, CDCl₃, TMS)
- δ =: 14,03 (s, OH), 8,82 (s, NH), 8,18-8,22 (2d, CH), 8,04-8,08 (d, CH), 7,82 (s, CH), 7,6-7,77 (m, CH), 6,9-7,36 (m, CH), 4,38-4,5 (t, CH₂), 4,02-4,15 (t, CH₂), 2,7-2,75 (s, CH₃), 1,9-1,97 (m, CH₂), 1,15-1,5 (m, CH₂, CH₃), 0,8-0,95 (m, CH₃)

Die nach den erfindungsgemäßen Verfahren herstelbaren Verbindungen der Formel I stellen wertvolle 2-Äquivalentcyankuppler dar und insbesondere Cyan-DIR-Kuppler, d.h. diese Verbindungen sind befähigt, bei der chromogenen Entwicklung einen blaugrünen Farbstoff zu bilden und gleichzeitig einen Entwicklungsinhiobitor in Freiheit zu setzen. Bevorzugte Beispiele der nach dem erfindungsgemäßen Verfahren hergestellten Verbindungen entsprechen der allgemeinen Formel Ia 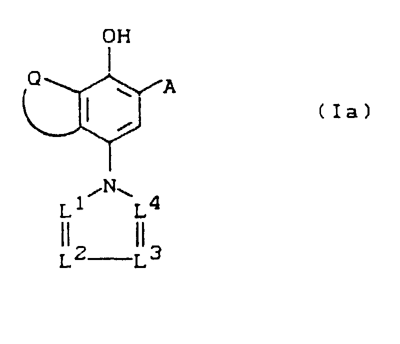 worin

A und Q die angegebene Bedeutung haben und worin 2 der Ringglieder (L¹, L², L³, L⁴) für ein N-Atom und die beiden anderen für eine Gruppe 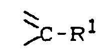 stehen, worin R¹ Alkyl, Alkylthio, Aryl, eine heterocyclische Gruppe (z.B. Furan oder Thiazol) oder -COOR² (mit R² = Alkyl oder Aryl) bedeutet. Diese Verbindungen sind neu.

## Patentansprüche

1. Verfahren zur Herstellung einer Verbindung der Formel I 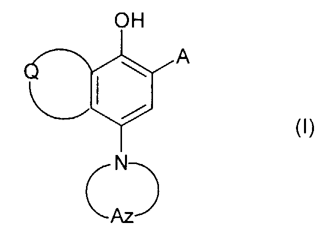 worin bedeuten
A eine Carbamoylgruppe, eine Sulfamoylgruppe oder eine Acylaminogruppe, die Ballastgruppen wie beispielsweise langkettige Alkylreste und/oder Phenyl- oder Phenoxygruppen enthalten können, die ihrerseits beispielsweise mit Alkyl-, Alkoxy-, Alkoxycarbonyl-, Alkylamino-, Acylamino-, Sulfonamido-, Carbamoyl- und/oder Sulfamoylgruppen substituiert sein können;
Az einen Rest zur Vervollständigung eines 5-gliedrigen heteroaromatischen Ringes (Azolring), an den ein 5- oder 6-gliedriger carbocyclischer oder heterocyclischer Ring ankondensiert sein kann;
Q einen Rest zur Vervollständigung eines ankondensierten Benzol- oder Pyridinringes, der beispielsweise durch Acylamino-, Sulfonamido-, Alkoxycarbonylamino-, Amino- oder Hydroxygruppen substituiert sein kann,
dadurch gekennzeichnet, daß eine Verbindung der Formel II 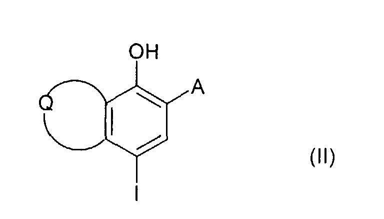 worin
A und Q die angegebene Bedeutung haben,
mit einem Acyloxygruppen übertragenden Oxidationsmittel oxidiert und anschließend mit einer Verbindung der Formel III umgesetzt wird 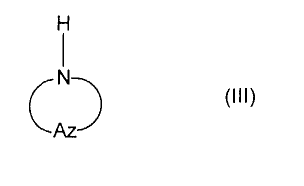 worin
Az die angegebene Bedeutung hat.

2. Verbindungen der Formel Ia 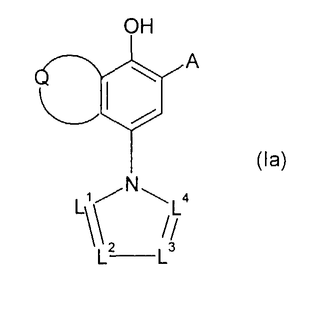 worin bedeuten
A eine Carbamoylgruppe, eine Sulfamoylgruppe oder eine Acylaminogruppe, die Ballastgruppen wie beispielsweise langkettige Alkylreste und/oder Phenyl- oder Phenoxygruppen enthalten können, die ihrerseits beispielsweise mit Alkyl-, Alkoxy-, Alkoxycarbonyl-, Alkylamino-, Acylamino-, Sulfonamido-, Carbamoyl- und/oder Sulfamoylgruppen substituiert sein können;
Q einen Rest zur Vervollständigung eines ankondensierten Benzol- oder Pyridinringes, der beispielsweise durch Acylamino-, Sulfonamido-, Alkoxycarbonylamino-, Amino- oder Hydroxygruppen substituiert sein kann;
L¹, L², L³, L⁴ Ringglieder mit der Maßgabe, daß zwei der Ringglieder für je ein N-Atom und die beiden anderen für je eine Gruppe 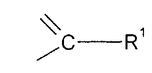 stehen, worin R¹ H, Alkyl, Alkylthio, Aryl, eine heterocyclische Gruppe oder -COOR² (mit R² = Aslkyl oder Aryl) bedeutet.

## Claims

1. Process for the production of a compound of the formula I 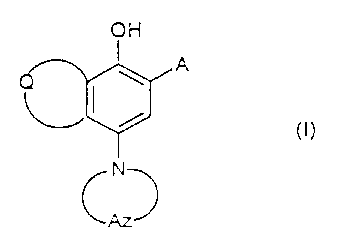 in which
A means a carbamoyl group, a sulfamoyl group or an acylamino group, which may contain ballast groups such as, for example, long-chain alkyl residues and/or phenyl or phenoxy groups, which may in turn be substituted with, for example, alkyl, alkoxy, alkoxycarbonyl, alkylamino, acylamino, sulfonamido, carbamoyl and/or sulfamoyl groups;
Az means a residue to complete a 5-membered heteroaromatic ring (azole ring), onto which a 5-or 6-membered carbocyclic or heterocyclic ring may be fused;
Q means a residue to complete a fused benzene or pyridine ring, which may be substituted by, for example, acylamino, sulfonamido, alkoxycarbonylamino, amino or hydroxy groups,
characterised in that a compound of the formula II 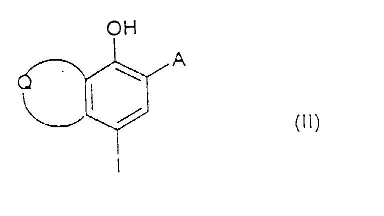 in which
A and Q have the stated meaning,
is oxidised with an acyloxy group-transferring oxidising agent and is then reacted with a compound of the formula III 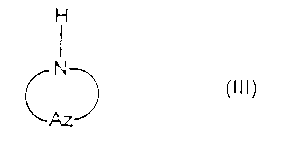 in which
Az has the stated meaning.

2. Compounds of the formula Ia 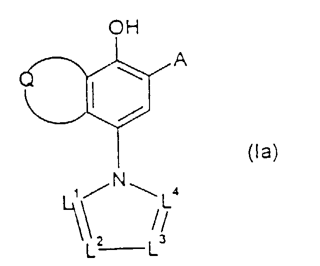 in which
A means a carbamoyl group, a sulfamoyl group or an acylamino group, which may contain ballast groups such as for example long-chain alkyl residues and/or phenyl or phenoxy groups, which may in turn be substituted with, for example, alkyl, alkoxy, alkoxycarbonyl, alkylamino, acylamino, sulfonamido, carbamoyl and/or sulfamoyl groups;
Q means a residue to complete a fused benzene or pyridine ring, which may be substituted by, for example, acylaminc, sulfonamido, alkoxycarbonylamino, amino or hydroxy groups;
L¹, L², L³, L⁴ mean ring members, with the proviso that two of the ring members each denote an N atom and the other two each denote a group 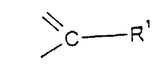 in which R¹ means H, alkyl, alkylthio, aryl, a heterocyclic group or -COOR² (where R² = alkyl or aryl).

## Revendications

1. Procédé de préparation d'un composé de formule I 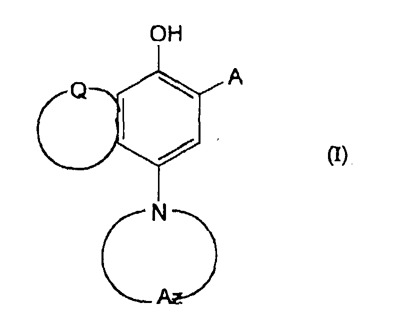 dans laquelle
A est un groupe carbamoyle, un groupe sulfamoyle ou un groupe acylamino, qui peut contenir des restes inertes, comme par exemple des restes alkyle à chaîne longue et/ou des groupe phényle ou phénoxy, qui de leur côté peuvent être substitués par exemple par des groupe alkyle, alcoxy, alcoxycarbonyle, alkylamino, acylamino, sulfonamido, carbamoyle et/ou sulfamoyle ;
Az est un reste pour compléter un cycle hétéroaromatique à 5 chaînons (cycle azole), sur lequel peut être condensé un cycle carbocyclique ou hétérocyclique à 5 ou 6 chaînons ;
Q est un reste pour compléter un cycle benzène ou pyridine condensé, qui peut être substitué par exemple par des groupes acylamino, sulfonamido, alcoxycarbonylamino, amino ou hydroxyle ;
caractérisé en ce qu'un composé de formule II 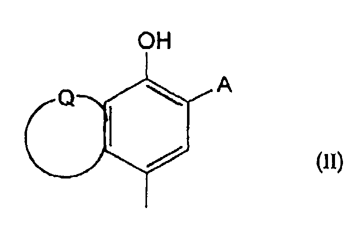 dans laquelle
A et Q ont les significations mentionnées ci-dessus,
est oxydé avec un moyen d'oxydation portant des groupes acyloxy et finalement est mis à réagir avec un composé de formule III 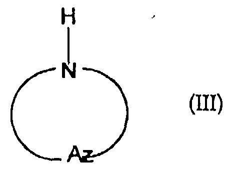 dans laquelle
Az a la signification mentionnée ci-dessus.

2. Composé de formule Ia 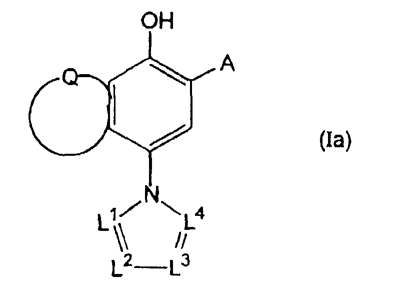 dans laquelle
A est un groupe carbamoyle, un groupe sulfamoyle ou un groupe acylamino, qui peut contenir des restes inertes, comme par exemple des restes alkyle à chaîne longue et/ou des groupe phényle ou phénoxy, qui de leur côté peuvent être substitués par exemple par des groupe alkyle, alcoxy, alcoxycarbonyle, alkylamino, acylamino, sulfonamido, carbamoyle et/ou sulfamoyle ;
Q est un reste pour compléter un cycle benzène ou pyridine condensé, qui peut être substitué par exemple par des groupes acylamino,sulfonamido, alcoxycarbonylamino, amino ou hydroxyle ;
L¹, L², L³, L⁴ représentent des chaînons de cycle avec la condition que deux des chaînon de cycle représentent chacun un atome de N et les deux autres représentent chacun un groupe 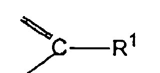 dans lequel R¹ est H, un groupe alkyle, alkylthio, aryle, un groupe hétérocyclique ou -COOR² (avec R² = alkyle ou aryle).
